# EUROPEAN PATENT SPECIFICATION

(11) **EP 1 392 103 B1**
(45) Date of publication and mention of the grant of the patent: **22.07.2009**
(21) Application number: 02721077.2
(22) Date of filing: 06.02.2002
(51) Int. Cl.: A01H 5/00, C12Q 1/68

(54) **METHODS OF MARKER-ASSISTED SELECTION OF HIGH OIL MAIZE PLANTS**
MARKER UNTERSTÜZTES VERFAHREN ZUR SELEKTION VON MAISPFLANZEN MIT ERHÖHTEM ÖLGEHALT
SELECTION ASSISTEE PAR MARQUEURS POUR OBTENIR PLANTES DE MAIS DONT LES GERMES POSSEDENT UNE TENEUR ELEVEE EN HUILE

(30) Priority: 06.02.2001 US 777798
(43) Date of publication of application: 03.03.2004
(73) Proprietor: Dow AgroSciences LLC, Indianapolis, Indiana 46268 (US)
(72) Inventor: THOMPSON, Steven, A., Carmel, IN 46033 (US); CUI, Cori, Carmel, IN 46032 (US); CLAYTON, Kathryn, Westfield, IN 46074 (US); REN, Ruihua, Westfield. IN 46074 (US); ERNST, Cynthia, Carmel, IN 46032 (US)
(74) Representative: Raynor, John
(86) International application number: PCT/US2002/005052
(87) International publication number: WO 2002/062129

(56) References cited:
- WO-A-98/42870
- US-A- 5 082 993
- US-A- 5 495 067
- US-A- 5 731 506
- US-A- 5 763 756
- US-A- 5 824 852
- US-A- 5 824 855
- US-A- 5 900 528
- US-A- 5 907 089
- SMITH, J.S.C., ET AL.: "an evaluation of the utility of SSR loci as molecular markers in maize (Zea mays L.): comparisons with data from RFLPs and pedigree" THEORETICAL AND APPLIED GENETICS, vol. 95, 1997, XP002308276
- BERKE T G ET AL: "QUANTITATIVE TRAIT LOCI FOR FLOWERING, PLANT AND EAR HEIGHT, AND KERNEL TRAITS IN MAIZE" CROP SCIENCE, CROP SCIENCE SOCIETY OF AMERICA, MADISON, WI, US, vol. 35, November 1995 (1995-11), pages 1542-1549, XP002070563 ISSN: 0011-183X
- GOLDMAN I L ET AL: "MOLECULAR MARKERS ASSOCIATED WITH MAIZE KERNEL OIL CONCENTRATION INAN ILLINOIS HIGH PROTEIN X ILLINOIS LOW PROTEIN CROSS" CROP SCIENCE, CROP SCIENCE SOCIETY OF AMERICA, MADISON, WI, US, vol. 34, July 1994 (1994-07), pages 908-915, XP002070562 ISSN: 0011-183X
- WATSON ET AL.: 'Breeding corn for increased oil content' PROCEEDINGS OF 3RD ANNUAL CORN & SORPHUM RESEARCH CONFERENCE vol. 30, 1975, pages 251 - 175, XP002956498

## Description

### Field of the Invention

The present invention relates to methods for genetic marker-assisted selection of maize plants utilizing genetic markers mapping to a genetic locus associated with high kernel oil content, and maize plants produced by such methods.

### Description of the Related Art

As further background, maize, often referred to in the United States as corn, has long been the subject of plant breeding. Maize can be bred by self-pollination and cross-pollination techniques, both of which are typically employed in plant breeding programs with the object of combining a set of desired traits in a single maize hybrid.

Maize plants which have been self-pollinated and selected for type for several generations become homozygous at almost all gene loci. Such maize plants produce a uniform population of true breeding progeny, often referred to as an inbred maize line. In turn, these inbred maize lines are used primarily in cross-pollination with other inbred maize lines, to produce hybrid maize seed for commercial sale.

In general, the development of a hybrid maize variety involves three steps. Plants from germplasm pools or breeding populations are first selected. The selected plants are then self-pollinated, or "selfed", for several generations and selected to produce a series of true-breeding inbred lines differing from one another. Finally, the inbred lines are crossed with other inbred lines to produce hybrid progeny, (F₁).

It is known that during the maize inbreeding process, the vigor of the maize line generally decreases. This vigor is then restored when two different inbred lines are crossed to produce the hybrid progeny (F₁). Importantly, a hybrid between a defined pair of inbred lines will always be the same, due to the homozygosity and homogeneity of the inbred line utilized. Thus, hybrid maize seed can be sold to the market which yields a reliable, reproducible maize plant.

One maize trait of interest is kernel oil content. Standard maize grain typically contains about 3 to 4% oil on a dry weight basis. Maize grain having an elevated oil concentration, referred to as "high oil maize", has a higher caloric content per unit weight and is thus particularly advantageous for use as animal feed, and for its reduced dusting during milling. Because more than half of the maize grain produced in the United States is used for animal feed, the use of high oil maize for feed is of substantial commercial interest. This is particularly so in view of the high caloric demands in feeding poultry (*Gallus gallus domesticus*), swine (*Sus scrofa*)*,* and dairy cows *(Bos* taurus). As it is, broiler and swine diets regularly contain added oil. Further, the full potential of bovine growth hormone in dairy cows will be more readily exploited if caloric intake is increased beyond that encountered with regular maize rations. Similar advantages may be expected in swine treated with porcine growth hormone.

Despite the expected advantages of high oil maize hybrids, the commercialization of high oil maize has been slow in progressing. This may be due to the fact that in several prior studies, breeding for high oil content has reduced yield both of inbreds and of hybrids made using the inbreds. Thus, competitive high-oil hybrids have proven difficult to develop.

In light of this background, a need exists for unique and effective methods for genetic marker-assisted maize selection and breeding.

### SUMMARY OF THE INVENTION

According to the present invention, a method of genetic marker-assisted selection of high oil level in maize plants is provided, comprising: isolating genomic DNA from the maize plants; assessing the DNA for the presence of SSR genetic markers phi-065 and/or bmc-1730 and absence of SSR genetic marker phi-61 to identify plants having a high oil trait locus on chromosome 9, that controls more than 50% of kernel oil variation and is mapped by bmc-1730 and/or phi-065, and lacking a waxy locus of which phi-061 forms part; and selecting the plants having said high oil trait locus and lacking said waxy locus.

The phi-061 marker is defined by a forward primer sequence as set forth in SEQ ID No. 1 and reverse primer sequence as set forth in SEQ ID No. 2. The bmc-1730 marker is defined by a forward primer sequence as set forth in SEQ ID No. 5 and reverse primer sequence as set forth in SEQ ID No. 6. The phi-065 marker is defined by a forward primer sequence as set forth in SEQ II7 No. 3 and reverse primer sequence as set forth in SEQ ID No. 4.

In preferred embodiments, the high oil trait locus on chromosome 9 is mapped by the genetic marker bmc-1730 or by both bmc-1730 and phi 065.

Additional embodiments, features and advantages of the present invention will be apparent to the skilled artisan upon reviewing the descriptions herein.

### DESCRIPTION OF THE PREFERRED EMBODIMENTS

The present invention provides methods as described above for genetic marker-assisted selection of maize plants, which involve the use of genetic markers which map to a locus on chromosome number 9 of inbred maize line 6UQ025, to select plants exhibiting a high kernel oil content.

As used herein, the term plant includes plant cells, plant protoplasts, plant cell tissue cultures from which maize plants can be regenerated, plant calli, plant clumps, and plant cells that are intact in plants or parts of plants, such as embryos, pollen, ovules, flowers, kernels, ears, cobs, leaves, husks, stalks, roots, root tips, anthers, silk and the like.

Inbred maize lines are typically developed for use in the production of maize hybrids and for use as germplasm in breeding populations for the creation of new and distinct inbred maize lines. Inbred maize lines are often used as targets for the introgression of novel traits through traditional breeding and/or molecular introgression techniques. Inbred maize lines need to be highly homogeneous, homozygous and reproducible to be useful as parents of commercial hybrids. Many analytical methods are available to determine the homozygosity and phenotypic stability of inbred lines.

The traditional analytical method is to observe phenotypic traits. The data are usually collected in field experiments over the life of the maize plants to be examined. Phenotypic characteristics most often observed include traits associated with plant morphology, ear and kernel morphology, insect and disease resistance, maturity, and yield.

Another trait that has been observed, particularly in the development of high oil maize, is kernel oil content. Such oil content has been measured using oil values both from composite samples and from single kernels. Silela et al., *Theor. Appl. Genet.,* 78:298 (1989), demonstrated that the rate of oil content gain was significantly higher if breeding selection occurred on a single kernel basis. In such methods, single kernel values may be measured utilizing wide-line nuclear magnetic resonance (NMR) spectroscopy to non-destructively determine the oil content of a single maize kernel. Experiments reported by Alexander et al., J. Am. Oil Chem. Soc., 44:555 (1967), demonstrated a highly positive correlation between NMR and solvent-extracted oil content determinations in maize. As well, Orman et al., J. Am. Oil Chem. Soc., 69:1036-1,038 (1992), demonstrated that near-infrared transmission spectroscopy (NITS) is useful to predict the oil content of single maize kernels. Accordingly, a variety of techniques such as these can be employed to observe kernel oil content in maize plants.

In addition to phenotypic observations, the genotype of a plant can also be examined. A variety of laboratory-based techniques are available for the characterization and comparison of plant genotypes as well. Among those commonly employed are Restriction Fragment Length Polymorphisms (RFLPs), Isozyme Electrophoresis, Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), Amplified Fragment Length Polymorphisms (AFLPs), and Simple Sequence Repeats (SSRs).

Upon extensive observation, inbred maize line 6UQ025 has shown uniformity and stability within the limits of environmental influence for typically assessed traits. The inbred has been self-pollinated for a sufficient number of generations with careful attention paid to uniformity of plant type to ensure the homozygosity and phenotypic stability necessary for use in commercial production.

Inbred maize line 6UQ025 is a yellow dent maize line of a medium-tall plant stature, medium-high ear height, and has leaves that are green-yellow in color. 6UQ025 has a large multi-branched tassel that has a heavy rate of pollen shed. The tassel florets have glumes that are light green and anthers that are salmon-purple in color. Ear shoots of 6UQ025 plants have silks that are pale green. The cobs of 6UQ025 ears are red and the kernels are a pale bronze color. Although some variation may occur due to environmental and other like influences, plants of inbred line 6UQ025 typically have a kernel oil content of about 17% to about 24% on a dry weight basis.

In any cross, one of the parental lines involved may be preferred as the male and the other as the female, considering the phenotypic characteristics of the parental plants that affect reproduction. As illustrations, one of the parental lines may have a higher seed yield, one may shed pollen more advantageously, and one may have more desirable tassel or seed characteristics. Maize line 6UQ025 is suited as either male or a female in crosses for producing first generation F₁ maize hybrids.

Inbred maize line 6UQ025 may be used as a donor of a chromosome 9 high oil locus and other oil loci in the production of hybrid maize and/or in the conversion of other elite lines which will in turn be used in the production of hybrid maize. Inbred maize line 6UQ025 may be used in conversions of elite inbred maize lines and as an oil donor in new breeding populations aimed at deriving new and distinct inbred maize lines with oil expression. These lines containing the donated chromosome 9 oil locus exhibit good combining ability when crossed to other elite inbred maize lines to produce competitive hybrids with elevated oil. For example, inbred maize line 6UQ025 has been used as a donor in the conversion of elite inbred lines. Conversions containing the chromosome 9 oil locus can be crossed with other elite inbred maize lines to result in maize hybrids.

Illustratively, one such conversion was made by crossing 6UQ025 as the male parent with a proprietary U.S.-derived elite line as the female parent, and the ultimate conversion containing the high-oil chromosome 9 locus had an oil level of 13.7% (dry weight basis). This conversion was crossed with the inbred maize line 4SQ601 (see U.S. Patent No. 5,986,182 issued November 16, 1999) to result in a New Hybrid maize comparing favorably to known commercial hybrids, as illustrated in Table 1.

The New Hybrid described in Table 1 is expected to have an oil content in the range of 5% to 7% (dry weight), elevated relative to normal corn hybrids with 4.2% oil (dry weight). The performance of such normal corn hybrids is illustrated by the Mycogen, Pioneer and DeKalb check hybrids identified in Table 1. The New Hybrid is competitive with these check hybrids in yield and other agronomic characteristics.

Additional conversions have been made by crossing 6UQ025 with a proprietary South America-derived elite line having about 4.2% oil (dry weight), with the conversions having from about 8% to 11% oil (dry weight) while exhibiting a recovery of about 75% to 80% of the original elite line genome (see Table 2, far right-hand columns). These conversions have been crossed with proprietary elite lines to result in further hybrids, having advantageous characteristics (see Table 2, left-hand column).

All hybrids identified in Table 2 (containing the high-oil chromosome 9 locus) are expected to have elevated oil levels, whereas the hybrids in Table 3 represent the same genetic background but without the high-oil chromosome 9 locus. As the data in these Tables illustrate, the new high-oil hybrids (Table 2) are competitive in yield and agronomic characteristics when compared to the comparative hybrids (Table 3). It will be understood that these specific examples are illustrative and that other similarly performing high-oil conversions and hybrids can be derived using 6UQ025 as the donor of the high-oil chromosome 9 locus.

**TABLE 1**

| **PEDIGREE** | **YIELD** | **MOIST** | **Stalk Lodging** | **Root Lodging** | **Test Weight** | **Population** |
|---|---|---|---|---|---|---|
| | **Bu./ acre** | **%** | **%** | **%** | **Lbs./Bu.** | **x 1,000 plants per acre** |
| New Hybrid | 181.4 | 22.5 | 0.0 | 0.0 | 52.6 | 30.9 |
| Mycogen 2620 | 165.6 | 19.5 | 0.5 | 0.0 | 55.8 | 31.5 |
| Pioneer 35R57 | 175.0 | 19.6 | 0.0 | 0.0 | 56.0 | 31.4 |
| Dekalb DK580 | 169.0 | 20.0 | 0.0 | 0.0 | 55.3 | 31.2 |
| Pioneer 3489 | 175.2 | 21.0 | 0.0 | 0.0 | 56.0 | 30.7 |
| Mycogen 2722 | 197.4 | 21.3 | 0.0 | 0.0 | 54.5 | 30.5 |
| **Statistics** | | | | | | |
| LSD 0.05 | 14.1 | 0.8 | 1 | 0 | 0.4 | |
| CV | 10.8 | 4.6 | 2.3 | - | 42.7 | |
| R | 0.6 | 0.9 | 0.8 | 0 | 1 | |
| MSE | 17.6 | 1 | 1.2 | 0 | 0.2 | |

**TABLE 2**

| **HIGH OIL HYBRIDS HAVING CHROMOSOME #9 LOCUS** | | | | | |
|---|---|---|---|---|---|
| **Hybrid** | **Dry Weight Yield** | **Moisture** **(%)** | **Lodging Score** | **% oil of Converter Inbred*** | **% Recovery of Converted Inbred** |
| 1 | 9648.5 | 23.6 | 3.4 | 8.1 | 81.0 |
| 2 | 9725.3 | 25.3 | 2.8 | 8.4 | 80.7 |
| 3 | 9438.8 | 25.6 | 5.2 | 9.0 | 75.6 |
| 4 | 9246.2 | 24.7 | 3.4 | 9.0 | 80.5 |
| 5 | 8971.0 | 25.3 | 5.8 | 9.9 | 77.0 |
| 6 | 8857.5 | 24.7 | 1.6 | 11.0 | 76.0 |
| 7 | 8852.2 | 24.3 | 3.3 | 8.9 | 78.3 |
| 8 | 8714.4 | 26.1 | 2.7 | 8.6 | 78.2 |
| 9 | 8587.6 | 24.4 | 5.3 | 8.9 | 76.6 |
| 10 | 8611.6 | 26.4 | 4.7 | 10.5 | 75.8 |
| 11 | 8501.9 | 24.2 | 7.6 | 8.2 | 79.7 |

| | | | | | |
|---|---|---|---|---|---|
| *Recurrent Parent Oil% was 4.2% for all entries. | | | | | |

**TABLE 3**

| **COMPARATIVE HYBRID LACKING CHROMOSOME #9 LOCUS*** | | | |
|---|---|---|---|
| **Hybrid** | **Dry weight Yield** | **Moisture (%)** | **Lodging Score** |
| Non-Oil Hybrid 1 | 9842.4 | 23.5 | 3.2 |
| Non-Oil Hybrid 2 | 9581.2 | 24.4 | 2.0 |

| | | | |
|---|---|---|---|
| *Hybrids prepared with same parent (4.2% oil) as hybrids identified in Table 2. | | | |

In further work, inbred maize line 6UQ025 was used in a series of crosses and selections to derive an inbred maize line carrying the chromosome 9 high oil locus. This inbred was then used as a high oil donor line to introgress increased oil concentration into a proprietary, elite maize line having a kernel oil content of about 4% (dry weight). After a series of backcrosses to the elite line while selecting for markers for the high oil chromosome 9 locus, as well as the high oil phenotype, a number of plants were selected. These selections were used in further backcrossing and selfing. Selection was based on improved elite recovery, minimization of donor segments in the area of the high oil locus on chromosome 9 (as identified by bmc-1730, phi-061,and phi-065), and possession of the high oil and shiny phenotype. Final selections exhibited the characteristics shown in Table 4:

**TABLE 4**

| Plant | %Oil | Kernel Type | % Recovery of Converted Inbred |
|---|---|---|---|
| A | 6.5 | Shiny | 96 |
| B | 8.3 | Shiny | 96 |
| C | 8.5 | Shiny | 96 |
| D | 6.8 | Shiny | 95 |
| E | 6.0 | Shiny | 97 |

Plants A-E carry the chromosome 9 high oil locus and have elevated oil content, and at the same time exhibit recovery of essentially all of the remaining genome of the elite line. Plants A-E were assessed for and no longer carry the donor alleles for the SSR markers bmc-1730, phi-061, and phi-065. Nonetheless, these plants carry the high oil chromosome 9 locus based on the stringent selection practices and maintained high oil phenotype. Phi-061 is known to be intronic to the waxy (wx) locus on chromosome 9, which is associated with a dull (non-shiny) kernel. Accordingly, this work confirms the linear association, but distinct nature, of the high oil locus and the wx locus occurring on chromosome 9. It also exhibits one potential strategy in breeding high oil maize plants, in which plants, for example inbred maize lines or hybrid plants, are derived which carry the chromosome 9 high oil locus but lack the chromosome 9 wx locus. Potential marker patterns for such plants include the presence of markers for the high-oil locus which are not part of the wx locus, illustratively phi-065 and/or bmc-1730, and the absence of phi-061 or other markers that are part of the wx locus. In this regard, phi-027 and phi-022 represent other, known markers that are a part of the waxy locus. Further, the nucleotide sequence for the waxy gene (genomic sequence) is known and has been published. Kloesgen, R.B., Gierl, A., Schwarz-Sommer, Z.S. and Saedler H., Molecular analysis of the waxy locus of zea mays, Mol. Gen. Genet. 203, 237-244 (1986). Such information can of course be used to identify additional sequences which may serve as markers for the waxy locus.

Inbred maize line 6UQ025, being substantially homozygous, can be reproduced by planting seeds of the line, growing the resulting maize plants under self-pollinating or sib-pollinating conditions with adequate isolation, and harvesting the resulting seed, using techniques familiar to the agricultural arts.

Maize plants may also be regenerated from cells in culture (Gordon-Kamm et al., Transformation of Maize Cells and Regeneration of Fertile Transgenic Plants," The Plant Cell, V.2, 603-618 (1990), herein incorporated by reference). For such regeneration, somatic cells from a 6UQ025 plant may be obtained and cultured *in vitro* in a medium comprising an embryogenic-promoting hormone to result in callus organization. Such agents may include, for example, dicamba, 2,4-D, and the like. The cells are then transferred to a medium which includes a tissue organization-promoting hormone such as BAP, myoinositol, 2,4-D, ABA, NAA, IAA and/or 2IP. After observation of tissue organization, the cells can be subcultured onto a medium without the hormone to allow for shoot elongation or root development, for example stimulated by IBA. Finally, the resulting plantlets can be transferred to a minimal medium to provide for hardening of the plant. Such minimal medium may be, for example, Clark's media.

Maize tissue culture procedures are also described in Green and Rhodes, "Plant Regeneration in Tissue Culture of Maize", Maize for Biological Research (Plant Molecular Biology Association, Charlottesville, VA 1982, at 367-372) and in Duncan, et al., "The Production of Callus Capable of Plant Regeneration from Immature Embryos of Numerous Zea Mays Genotypes", 165 Planta 322-332 (1985). Thus, it is clear from the literature that the state of the art is such that regenerative methods of obtaining plants are routinely used.

The method of the present invention provides for genetic marker-assisted selection which can be used at any stage of population development to provide maize plants such as inbred lines having high kernel oil content. Such inbred lines can then be crossed with one or more different inbred line to produce hybrid seed from which high oil hybrid maize plants can be grown. A trait locus associated with high kernel oil content has been identified on chromosome 9, which is mapped by the SSR genetic markers phi-061, bmc-1730 and phi-065, defined by the primers set forth below:

| Marker | Primer Sequence (5'-3') | Direction | SEQ ID NO. |
|---|---|---|---|
| Phi-061 | GACGTAAGCCTAGCTCTGCCAT | forward | 1 |
| | AAACAAGAACGGCGGTGCTGATTC | reverse | 2 |
| Phi-065 | AGGGACAAATACGTGGAGACACAG | forward | 3 |
| | CGATCTGCACAAAGTGGAGTAGTC | reverse | 4 |
| Bmc-1730 | GGGTGCTCGTAGTAGGGGTT | forward | 5 |
| | AACACGTCAACAAGGGGAAG | reverse | 6 |

The high oil genetic locus mapped by these markers has been found to control more than 50% of the kernel oil variation in a population derived from 6UQ025. Thus, highly advantageous methods for the identification, breeding and development of high oil maize plants are provided, which include the assessment of maize plant DNA (e.g. genomic DNA) for the presence of such genetic locus, utilizing the aforesaid markers. Maize lines or other substantially homogeneous maize populations that include a high oil genetic locus mapped by these SSR markers can be produced, for example, in breeding techniques utilizing inbred line 6UQ025 to provide the source of the high oil marker(s). Moreover, such plants can be utilized in further breeding in fashions similar to those described above for 6UQ025.

The newly identified high oil locus on chromosome 9 is linearly associated with the waxy (wx) locus on chromosome 9, but is separate therefrom. Of the three above-identified SSR markers found to map to the high oil locus, one, phi-061, is known to be intronic to the wx locus. Thus, to produce high oil-positive, wx-negative plants a breeding program can be undertaken selecting for the high oil locus and against the wx locus by selecting plants that are phi-065-positive and/or bmc-1730-positive, and phi-061-negative.

Maize is used as human food, livestock feed, and as raw material in industry. The food uses of maize, in addition to human consumption of maize kernels, include both products of dry-milling and wet-milling industries. The principal products of maize dry milling are grits, meal and flour. The maize wet-milling industry can provide maize starch, maize syrups, and dextrose for food use. Maize oil is recovered from maize germ, which is a by-product of both dry- and wet-milling industries.

Maize, including both grain and non-grain portions of the plant, is also used extensively as livestock feed, primarily for beef cattle, dairy cattle, hogs, and poultry. For these and many other applications, a component of the manufacturing process involves the separation of the grain of the plant from the cob. Such processes are also a part of the present invention.

Industrial uses of maize include production of ethanol, maize starch in the wet-milling industry and maize flour in the dry-milling industry. The industrial applications of maize starch and flour are based on functional properties, such as viscosity, film formation, adhesive properties, and ability to suspend particles. The maize starch and flour have applications in the paper and textile industries. Other industrial uses include applications in adhesives, building materials, foundry binders, laundry starches, explosives, oil-well muds, and other mining applications.

Plant parts other than the grain of maize are also used in industry. For example, stalks and husks are made into paper and wallboard and cobs are used for fuel and to make charcoal.

The seeds of inbred maize line 6UQ025, the plants produced from the inbred seeds, the hybrid maize plants produced from the crossing of the inbreds, hybrid seeds, and various parts of the hybrid maize plants can be utilized for human food, livestock feed, and as raw materials in industry.

Applicants have made a deposit of at least 2500 seeds of Inbred Corn Line 6UQ025 with the American Type Culture Collection (ATCC), Rockville, MD 20852 U.S.A. The seeds were deposited with the ATCC on October 19, 2000 under Accession No. PTA-2607, and were taken from samples maintained by Agrigenetics dba Mycogen Seeds, located at the research facility near Windfall, Indiana, USA from a date prior to the filing date of this application. The deposit of the Inbred Corn Line 6UQ025 will be maintained in the ATCC depository, which is a public depository, for a period of 30 years, or 5 years after the most recent request, or for the enforceable life of the patent, whichever is longer; and will be replaced if it becomes nonviable during that period. Additionally, Applicants have satisfied all the requirements of 37 C.F.R. §§1.801-1.809, including providing an indication of the viability of the sample. Applicants impose no restrictions on the availability of the deposited material from the ATCC; however, Applicants have no authority to waive any restrictions imposed by law on the transfer of biological material or its transportation in commerce. Applicants do not waive any infringement of its rights granted under this patent or under the Plant Variety Protection Act (7 U.S.C. 2321 et seq.).

## Claims

1. A method of genetic marker-assisted selection of high oil level in maize plants, comprising:
isolating genomic DNA from the maize plants;
assessing the DNA for the presence of SSR genetic markers phi-065 and/or bmc-1730 and absence of SSR genetic marker phi-61 to identify plants having a high oil trait locus on chromosome 9, that controls more than 50% of kernel oil variation and is mapped by bmc-1730 and/or phi-065, and lacking a waxy locus of which phi-061 forms part, wherein: the phi-061 marker is defined by a forward primer sequence as set forth in SEQ ID No. 1 and reverse primer sequence as set forth in SEQ ID No. 2; the bmc-1730 marker is defined by a forward primer sequence as set forth in SEQ ID No. 5 and reverse primer sequence as set forth in SEQ ID No. 6; and the phi-065 marker is defined by a forward primer sequence as set forth in SEQ ID No. 3 and reverse primer sequence as set forth in SEQ ID No. 4; and
selecting the plants having said high oil trait locus and lacking said waxy locus.

2. The method of claim 1, wherein the high oil trait locus on chromosome 9 is mapped by the genetic marker brine-1730.

3. The method of claim 1, wherein the high oil trait locus on chromosome 9 is mapped by the genetic marker bmc-1730 and phi 065.

## Patentansprüche

1. Durch einen genetischen Marker unterstütztes Verfahren zur Selektion von Maispflanzen mit erhöhtem Ölgehalt, umfassend:
Isolieren genomischer DNA aus den Maispflanzen;
Abschätzen der DNA hinsichtlich der Anwesenheit der genetischen SSR-Marker phi-065 und/oder bmc-1730 und der Abwesenheit des genetischen SSR-Markers phi-061, um Pflanzen zu identifizieren, die einen Locus mit dem Merkmal viel Öl auf Chromosom 9 haben, der mehr als 50% der Kernöl-Variation kontrolliert und von bmc-1730 und/oder phi-065 kartiert ist, und die keinen waxy (wx) Locus haben, von dem phi-061 einen Teil bildet, worin: der Marker phi-061 durch einen Vorwärtsprimer mit der Sequenz wie in der SEQ ID Nr.: 1 dargelegt und einen Rückwärtsprimer mit der Sequenz wie in der SEQ ID Nr.: 2 dargelegt, definiert ist; der Marker bmc-1730 durch einen Vorwärtsprimer mit der Sequenz wie in der SEQ ID Nr.: 5 dargelegt und einen Rückwärtsprimer mit der Sequenz wie in der SEQ ID Nr.: 6 dargelegt, definiert ist und der Marker phi-065 durch einen Vorwärtsprimer mit der Sequenz wie in der SEQ ID Nr.: 3 dargelegt und einen Rückwärtsprimer mit der Sequenz wie in der SEQ ID Nr.: 4 dargelegt, definiert ist; und
Selektieren der Pflanzen, die den Locus mit dem Merkmal viel Öl haben und keinen waxy (wx) Locus.

2. Verfahren nach Anspruch 1, worin der Locus mit dem Merkmal viel Öl auf Chromosom 9 durch den genetischen Marker bmc-1730 kartiert ist.

3. Verfahren nach Anspruch 1, worin der Locus mit dem Merkmal viel Öl auf Chromosom 9 durch die genetischen Marker bmc-1730 und phi-065 kartiert ist.

## Revendications

1. Procédé de sélection, assistée par marqueurs génétiques, d'une haute teneur en huile chez des plants de maïs, comprenant les étapes suivantes :
- isoler de l'ADN génomique de plants de maïs ;
- tester chez cet ADN la présence des marqueurs génétiques SSR phi-065 et/ou bmc-1730 et l'absence du marqueur génétique SSR phi-061, afin d'identifier les plants qui possèdent sur le chromosome 9 un locus associé au caractère de haute teneur en huile, qui commande plus de 50 % de la variation de la teneur en huile des grains et qui est cartographié à l'aide de bmc-1730 et/ou phi-065, et auxquels il manque un locus associé au caractère cireux, duquel fait partie phi-061, étant entendu que :
le marqueur phi-061 est défini par la séquence amorce directe donnée en tant que Séquence N° 1 et la séquence amorce inverse donnée en tant que Séquence N° 2 ;
le marqueur bmc-1730 est défini par la séquence amorce directe donnée en tant que Séquence N° 5 et la séquence amorce inverse donnée en tant que Séquence N° 6 ;
et le marqueur phi-065 est défini par la séquence amorce directe donnée en tant que Séquence N° 3 et la séquence amorce inverse donnée en tant que Séquence N° 4 ;
- et sélectionner les plants qui possèdent ledit locus associé au caractère de haute teneur en huile et auxquels il manque ledit locus associé au caractère cireux.

2. Procédé conforme à la revendication 1, dans lequel ledit locus associé au caractère de haute teneur en huile, présent sur le chromosome 9, est cartographié à l'aide du marqueur génétique bmc-1730.

3. Procédé conforme à la revendication 1, dans lequel ledit locus associé au caractère de haute teneur en huile, présent sur le chromosome 9, est cartographié à l'aide des marqueurs génétiques bmc-1730 et phi-065.


### SEQUENCE LISTING

<110> DOW AGROSCIENCES LLC
   THOMPSON, STEVEN
   CUI, CORY
   CLAYTON, KATHRYN
   ERNST, CYNTHIA
   REN, RUIHUA
<120> HIGH OIL MAIZE PLANTS, AND METHODS FOR OBTAINING SAME
<130> PBA/P004461PEP
<160> 6
<170> PatentIn version 3.1
<210> 1
   <211> 22
   <212> DNA
   <213> Artificial
<220>
   <221> misc_feature
   <222> (1)..(22)
   <223> Forward Primer for Phi-061
<400> 1
   gacgtaagcc tagctctgcc at 22
<210> 2
   <211> 24
   <212> DNA
   <213> Artificial
<220>
   <221> misc_feature
   <222> (1)..(24)
   <223> Reverse primer for Phi-061
<400> 2
   aaacaagaac ggcggtgctg attc 24
<210> 3
   <211> 24
   <212> DNA
   <213> Artificial
<220>
   <221> misc_feature
   <222> (1)..(24)
   <223> Forward primer for Phi-065
<400> 3
   agggacaaat acgtggagac acag 24
<210> 4
   <211> 24
   <212> DNA
   <213> Artificial
<220>
   <221> misc_feature
   <222> (1)..(24)
   <223> Reverse primer for Phi-065
<400> 4
   cgatctgcac aaagtggagt agtc 24
<210> 5
   <211> 20
   <212> DNA
   <213> Artificial
<220>
   <221> misc_feature
   <222> (1)..(20)
   <223> Forward primer for Bmc-1730
<400> 5
   gggtgctcgt agtaggggtt 20
<210> 6
   <211> 20
   <212> DNA
   <213> Artificial
<220>
   <221> misc_feature
   <222> (1)..(20)
   <223> Reverse primer for Bmc-1730
<400> 6
   aacacgtcaa caaggggaag 20